# EUROPÄISCHE PATENTSCHRIFT

(11) **EP 1 474 051 B1**
(45) Veröffentlichungstag und Bekanntmachung des Hinweises auf die Patenterteilung: **27.07.2005**
(21) Anmeldenummer: 02709958.9
(22) Anmeldetag: 11.02.2002
(51) Int. Cl.: A61B 17/70

(54) **VORRICHTUNG ZUR VERBINDUNG EINES LÄNGSTRÄGERS MIT EINEM KNOCHEN**
DEVICE FOR LINKING A LONGITUDINAL SUPPORT WITH A BONE
DISPOSITIF POUR RELIER UN SUPPORT LONGITUDINAL A UN OS

(43) Veröffentlichungstag der Anmeldung: 10.11.2004
(73) Patentinhaber: SYNTHES AG CHUR, 7002 Chur (CH)
(72) Erfinder: DONATH, Raoul, 79639 Grenzach-Wyhlen (DE)
(74) Vertreter: Lusuardi, Werther Giovanni, Dr.
(86) Internationale Anmeldenummer: PCT/CH2002/000080
(87) Internationale Veröffentlichungsnummer: WO 2003/068086

(56) Entgegenhaltungen:
- US-A- 5 584 834
- US-A- 5 728 098
- US-B1- 6 254 602
- US-B1- 6 273 888

## Beschreibung

Die Erfindung bezieht sich auf eine Vorrichtung zur Verbindung eines Längsträgers mit einem Knochen, gemäss dem Oberbegriff des Patentanspruchs 1 sowie mit einem Implantat gemäss dem Oberbegriff des Patentanspruchs 8.

Bei internen Fixationen der Wirbelsäule oder Wirbelsäulenteilen werden vorzugsweise Vorrichtungen eingesetzt, welche Pedikelschrauben, mindestens einen Längsträger und Verbindungsteile zur Befestigung der Pedikelschrauben am Längsträger umfassen. Die Pedikelschrauben werden in die Pedikel der einzelnen zu verbindenden Wirbelkörper eingeschraubt und mit dem Schraubenkopf im Verbindungsteil befestigt. Zur stabilen Verankerung des gesamten Implantates müssen die Pedikelschrauben einerseits fest in den Pedikeln verankert sein und andererseits starr mit dem Längsträger verbindbar sein. Die Verbindung zwischen dem Schraubenkopf und dem Verbindungsteil erfolgt meistens über einen Klemmechanismus, welcher lösbar sein muss, damit das gesamte Implantat ohne grosse Gewebeöffnungen im Bereich der Wirbelsäule wieder entfernbar ist.

Eine Vorrichtung zur Verbindung einer Pedikelschraube mit einem Längsträger ist aus der US 5,728,098 SHERMAN bekannt. Diese bekannte Vorrichtung umfasst eine Pedikelschraube mit einem sphärischen Schraubenkopf und ein Verbindungsteil mit einem Hohlraum, welcher gegen das untere, an die Pedikelschraube angrenzende Ende ebenfalls sphärisch ausgestaltet ist und zur Aufnahme des Schraubenkopfes der Pedikelschraube dient. Am oberen Ende des Verbindungsteiles ist ein quer zur Längsachse des Verbindungsteiles angelegter Kanal zur Aufnahme eines Längsträgers angeordnet. Sowohl die beiden den Kanal begrenzenden Zungen, als auch das untere Segment mit dem sphärischen Hohlraum sind quer zur Längsachse elastisch deformierbar. Dadurch wird erreicht, dass einerseits nach dem Einlegen des Schraubenkopfes der Pedikelschraube das untere Segment des Verbindungsteiles quer zur Längsachse komprimiert werden kann und dadurch der Schraubenkopf im Hohlraum fixiert werden kann, als auch andererseits nach dem Einlegen des Längsträgers in den Kanal das obere Segment des Verbindungsteiles quer zur Längsachse komprimiert werden kann und dadurch der Längsträger im Kanal zwischen den Zungen fixiert werden kann. Das obere sowie das untere Segment des Verbindungsteiles werden mittels Klemmringen komprimiert, welche zur axialen Fixierung in Nuten am Verbindungsteil einrastbar sind. Nachteilig bei dieser bekannten Vorrichtung ist, dass der Klemmring, welcher zur Fixierung des Längsträgers im oberen Segment des Verbindungsteiles dient, nach dem Einführen des Längsträgers in den Kanal vom Chirurgen separat als Einzelteil durch die Weichteile zum Implantat gebracht werden muss, so dass ein weiterer Arbeitsschritt notwendig wird. Zudem wird die Gefahr des Verlierens des Klemmringes in den Weichteilen während der Implantation erhöht.

Hier will die Erfindung Abhilfe schaffen. Der Erfindung liegt die Aufgabe zugrunde, eine Vorrichtung zu schaffen, deren Klemmring vor der Implantation mit der Vorrichtung verbindbar ist und nach dem Einführen des Längsträgers die Blockierung der Vorrichtung durch einfaches Verschieben des Klemmringes erreichbar ist.

Die Erfindung löst die gestellte Aufgabe mit einer Vorrichtung zur Verbindung eines Längsträgers mit einem Knochen oder Knochenfragment, welche die Merkmale des Anspruchs 1 aufweist, sowie mit einem Implantat, welches die Merkmale des Anspruchs 8 aufweist.

Die erfindungsgemässe Vorrichtung umfasst im wesentlichen ein Knochenfixationsmittel mit einem Oberteil zur Verbindung mit einem Längsträger und einem Unterteil zur Fixation mit einem Knochen, insbesondere einem Pedikel. Der Längsträger ist in einen Kanal mit quer zur Zentralachse des Knochenfixationsmittels verlaufender Kanalachse einlegbar. Mittels eines Klemmringes, welcher eine zur Zentralachse koaxiale Zentralbohrung umfasst und am Oberteil unterhalb des Kanals in eine erste axiale Position bringbar ist, bei welcher ein im Kanal eingelegter Längsträger relativ zum Knochenfixationsmittel blockierbar ist.

Das Oberteil ist mittels mindestens einem zwischen seinem unteren Ende und dem Kanal parallel zur Zentralachse angeordneten Schlitz quer zur Zentralachse elastisch deformierbar ausgestaltet. Durch den Kanal und den Schlitz, welche beide das Oberteil quer zur Zentralachse durchdringen, werden am Oberteil mindestens zwei quer zur Kanalachse elastisch deformierbare Zungen gebildet. Femer umfasst das Oberteil eine zur Zentralachse konzentrische, kreisförmige Nute mit einem Kerndurchmesser, welcher kleiner als der Durchmesser der Zentralbohrung am Klemmring ist, so dass bei einer zweiten, axial mit der Nute zusammenfallenden Position des Klemmringes einerseits die beiden durch den Kanal und den mindestens einen Schlitz gebildeten, elastisch quer zur Zentralachse deformierbaren Zungen am Oberteil des Knochenfixationsmittels quer zur Kanalachse elastisch deformierbar sind und ein Einlegen des Längsträgers in den Kanal ermöglicht wird und andererseits nach dem Einbringen des Längsträgers in den Kanal Repositionsmanöver am zu stabilisierenden Knochen, beispielsweise der zu stabilisierenden Wirbelsäulenteile durchgeführt werden können.

Der Längsträger wird während des Reponierens der zu stabilisierenden Knochen durch die beiden elastisch deformierbaren Zungen leicht arretiert, so dass Verschiebungen oder Rotationsbewegungen des Längsträgers im Kanal gegen einen Widerstand möglich sind.

Die durch die Erfindung erreichten Vorteile sind im wesentlichen darin zu sehen, dass dank der erfindungsgemässen Vorrichtung:
- das vormontierte Implantat ohne Gefahr des Verlierens von Einzelteilen implantierbar ist; und
- eine Position des Klemmechanismus für die Reposition beinhaltet.

In der bevorzugten Ausführungsform der erfindungsgemässen Vorrichtung umfasst diese ein Oberteil, welches zwischen der Nute und dem unteren Ende einen Durchmesser D aufweist, welcher grösser oder gleich dem Durchmesser d der Zentralbohrung im Klemmring ist. Das Verhältnis zwischen dem Durchmesser D und dem Durchmesser d beträgt zwischen 102% und 100%, vorzugsweise zwischen 100,5% und 100%. Damit ist der Vorteil erreichbar, dass der Klemmring bei der Vormontage des Implantates vom unteren Ende des Oberteils bis zur Nute über dieses schiebbar oder pressbar ist, sich jedoch nicht von selbst aus der Nute heraus über das untere Ende des Oberteils verschieben kann. Diese Eigenschaft kann noch durch die Ausgestaltung der Zentralbohrung im Klemmring mit einer konischen oder sphärischen Erweiterung am oberen Ende der Zentralbohrung und einer scharfen Kante am unteren Ende der Zentralbohrung verstärkt werden.

In einer anderen Ausführungsform der erfindungsgemässen Vorrichtung umfasst diese zwischen dem Kanal und der Nute einen zur Zentralachse konzentrischen, kreisförmigen Absatz. Das Verhältnis des Durchmessers dieses Absatzes zum Durchmesser der Zentralbohrung im Klemmring beträgt zwischen 101% und 110%, vorzugsweise zwischen 101% und 105%. Damit ist der Vorteil erreichbar, dass sich der Klemmring nach der Fixation des Längsträgers im Kanal nicht von selbst axial über die Nute verschieben kann und sich die Blockierung der Vorrichtung lösen könnte.

In einer weiteren Ausführungsform der erfindungsgemässen Vorrichtung umfasst diese zwischen dem Absatz und dem Kanal eine zweite Nute, worin der Klemmring zur Blockierung des im Kanal eingelegten Längsträgers einrastbar ist. Das Verhältnis des Kerndurchmessers D_{K2} dieser zweiten Nute zum Durchmesser d der Zentralbohrung im Klemmring beträgt zwischen 102% und 100%, vorzugsweise zwischen 100,5% und 100%. Damit ist der Vorteil erreichbar, dass der Klemmring in seiner ersten Position axial in der Nute arretierbar ist.

Vorzugsweise ist das Unterteil des Knochenfixationsmittels als Pedikelschraube mit einem ein Aussengewinde aufweisenden Schraubenschaft ausgestattet. In anderen Ausführungsformen ist das untere Segment des Knochenfixationsmittels als Pedikelhaken ausgeführt.

Das erfindungsgemässe Implantat umfasst neben dem Knochenfixationsmittel einen stabförmigen Längsträger mit einem Aussendurchmesser D_{L}. Der Kanal weist dabei eine Licht Weite L_{W} auf, welche zum Durchmesser D_{L} in einem Verhältnis L_{W} / D_{L} zwischen 90% und 105%, vorzugsweise zwischen 98% und 102% steht. Damit lässt sich der Vorteil erreichen, dass der Längsträger im nicht blockierten Zustand der Vorrichtung durch die Zungen am Oberteil des Knochenfixationsmittels elastisch arretiert wird und somit Repositionsmanöver an den Knochenteile, insbesondere den Wirbelsäulenteilen ausführbar sind und der Längsträger dennoch leicht festgeklemmt wird.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Ansicht einer Ausführungsform der erfindungsgemässen Vorrichtung mit blockiertem Längsträger;
Fig. 2 eine Ansicht der in Fig. 1 dargestellten Ausführungsform der erfindungsgemässen Vorrichtung, wobei sich der Klemmring in der zur Reposition von Knochenteilen vorgesehenen Position befindet;
Fig. 3 ein Aufsicht auf die in den Fig. 1 und 2 dargestellte Ausführungsform der erfindungsgemässen Vorrichtung mit eingelegtem Längsträger;
Fig. 4 einen Ausschnitt aus dem Oberteil einer Ausführungsform der erfindungsgemässen Vorrichtung; und
Fig. 5 eine Ansicht einer Ausführungsform des erfindungsgemässen Implantates, welches an einem Wirbelsäulenteil angeschraubt ist.

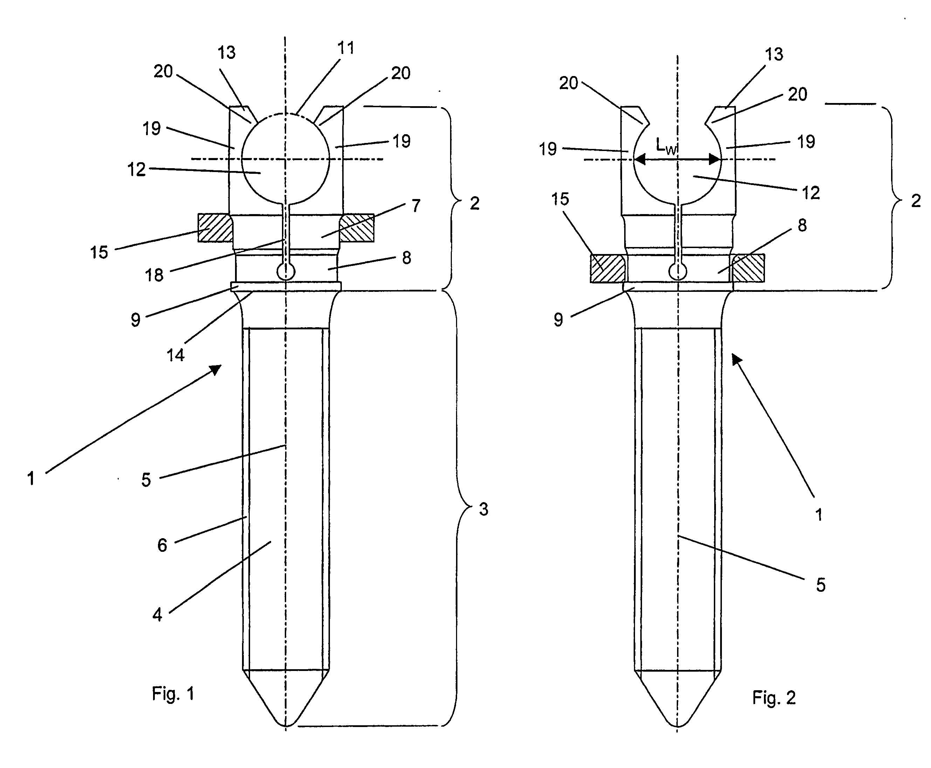

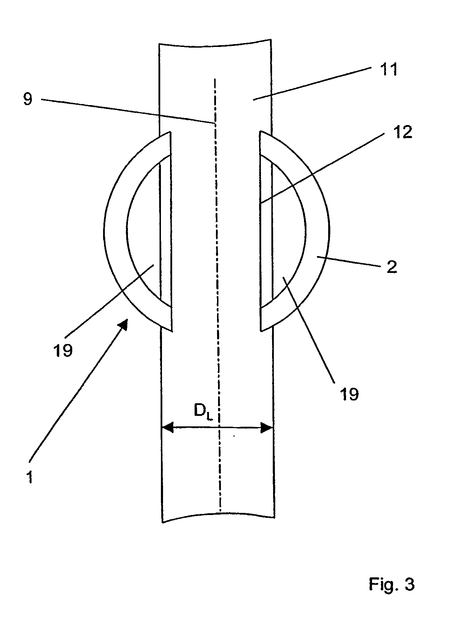

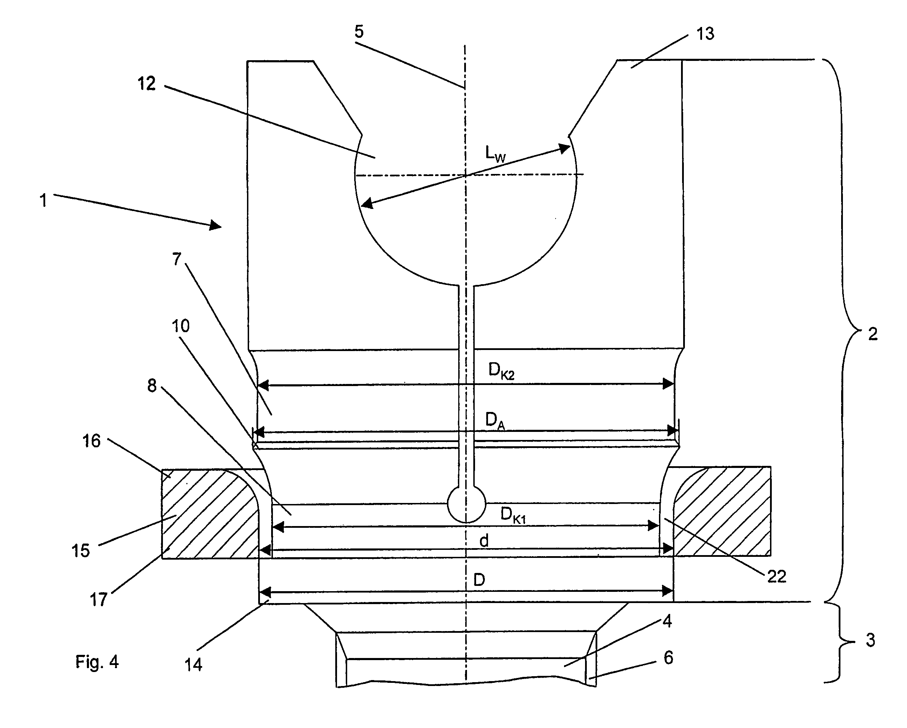

Fig. 1, 2 und 3 zeigen ein Knochenfixationsmittel 1 mit einem Oberteil 2 und einem Unterteil 3, welche zur einer Zentralachse 5 koaxial angeordnet sind. Das Unterteil 3 ist als Gewindeschaft 4 mit einem Aussengewinde 6 zum Einschrauben in einen Pedikel eines Wirbelkörpers 24 (Fig. 4) ausgestaltet. Das Oberteil 2 ist als Verbindungsteil zur Verbindung des Knochenfixationsmittel 1 mit einem Längsträger 11 ausgestaltet, wobei der zur Aufnahme des Längsträgers 11 dienende Kanal 12 das Oberteil 2 quer zur Zentralachse 5 durchdringt und am oberen Ende 13 des Knochenfixationsmittels 1 offen ist. Zwischen dem unteren Ende 14 des Oberteils 2 und dem Kanal 12 ist am Oberteil 2 ein Schlitz 18 angebracht, welcher das Oberteil 2 parallel zur Kanalachse 9 durchdringt und gegen den Kanal 12 offen ist. Durch den Kanal 12 und den Schlitz 18 werden am Oberteil 2 zwei zur Zentralachse 5 parallele Zungen 19 gebildet, welche quer zur Kanalachse 9 elastisch deformierbar sind. Der Kanal 12 weist eine Lichte Weite L_{W} auf und wird am oberen Ende 13 des Knochenfixationsmittels 1 durch zwei Nocken 20 quer zur Zentralachse 5 verengt, so dass ein den Kanal 12 eingelegter Längsträger 11 durch die Nocken 20 axial gegen das obere Ende 13 des Knochenfixationsmittels 1 arretiert wird. Bei der Implantation des Längsträgers 11 wird dieser parallel zur Zentralachse 5 vom oberen Ende 13 des Knochenfixationsmittels 1 her zwischen den Nocken 20 hindurch in den Kanal 12 verschoben, wobei die beiden elastischen Zungen 19 quer zur Zentralachse 5 elastisch gespreizt werden, wenn der Längsträger 11 an den Nocken 20 vorbei geschoben wird. Sobald der Längsträger 11 vollständig in den Kanal 12 geschoben ist, federn die Zungen 19 zurück in ihre nicht deformierte Position. Zum Einbringen des Längsträgers 11 in den Kanal 12 ist der Klemmring 15 axial über die zur Zentralachse 5 konzentrische, kreisförmige Nute 8 geschoben (Fig. 2), deren Kerndurchmesser D_{K1} (Fig. 3) kleiner als der Durchmesser d (Fig. 3) der Zentralbohrung 22 im Klemmring 15 ist, so dass die beiden Zungen 19 ohne Entfernen des Klemmringes 15 vom Oberteil 2 quer zur Zentralachse 5 spreizbar sind. Nach dem Einbringen des Längsträgers 11 in den Kanal 12 wird der Klemmring 15 in die zweite Nute 7 axial verschoben. Der Kerndurchmesser D_{K2} der Nute 7 ist so bemessen, dass in dieser Position des Klemmringes 15 der Längsträger 11 im Kanal 12 blockiert wird (Fig. 1).

Fig. 4 zeigt das Oberteil 2 einer Ausführungsform der erfindungsgemässen Vorrichtung mit einer ersten, einen Kerndurchmesser D_{K1} < d aufweisenden Nute 8 und einer zweiten, einen Kerndurchmesser D_{K2} ≥ d aufweisenden Nute 7. Zum Einlegen des Längsträgers 11 (Fig. 3) in den Kanal 12 und während des Reponierens der zu stabilisierenden Knochen wird der Klemmring 15 in die erste Nute 8 in seine zweite Position geschoben. Durch axiales Verschieben des Klemmringes 15 gegen das obere Ende 13 des Oberteils 2 ist der Klemmring 15 in die zweite Nute 7 bringbar. In dieser ersten Position des Klemmringes 15 wird der Längsträger 11 (Fig. 3) im Kanal 15 blockiert. Der Kerndurchmesser D_{K2} der zweiten Nute 7 ist kleiner als der Durchmesser des Oberteils 2 zwischen der zweiten Nute 7 und dem Kanal 15. Zwischen den beiden Nuten 7;8 ist ein zur Zentralachse 5 konzentrischer, kreisförmiger Absatz 10 mit dem Durchmesser D_{A} > d angeordnet, so dass der Klemmring 15 in seiner ersten Position axial arretiert ist. Am seinem unteren Ende 14 weist das Oberteil 2 einen Durchmesser D auf, welcher so bemessen ist, dass der Klemmring 15 während der Vormontage der Vorrichtung vom Unterteil 3 her in die Nute 8 bringbar ist, jedoch gegen ein unbeabsichtigtes Verschieben von der Nute 8 gegen das Unterteil 3 gesichert ist. Der Übergang zwischen der Nute 8 und dem unteren Ende 14 des Oberteils 2 ist scharfkantig ausgestaltet. Ebenfalls scharfkantig ausgestaltet ist die Mündung der Zentralbohrung 22, so dass durch die beiden scharfen Kanten ein unbeabsichtigtes Verschieben des Klemmringes 15 von der Nute 8 gegen das untere Ende 14 des Oberteils 2 zusätzlich behindert wird. Die Verschiebung des Klemmringes 15 von der ersten Nute 8 in die zweite Nute 7 wird durch eine sphärische Erweiterung der Zentralbohrung 22 am oberen Ende 16 des Klemmringes 15 sowie durch einen ebenfalls gekrümmten Übergang zwischen der ersten Nute 8 und dem Absatz 10 begünstigt.

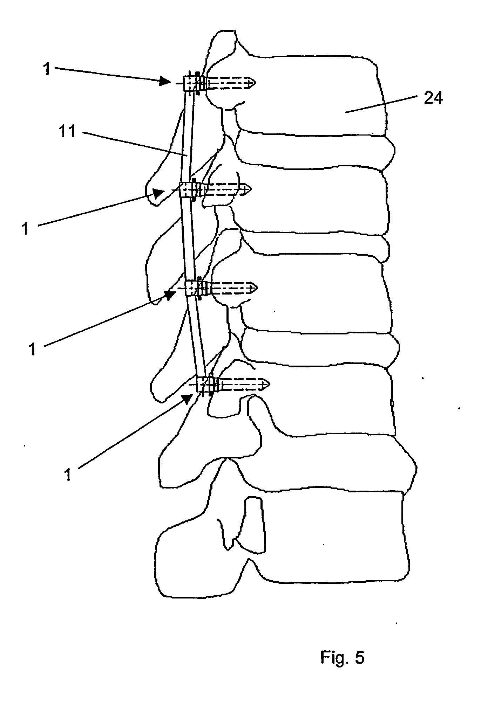

Fig. 5 zeigt das erfindungsgemässe Implantat bestehend aus vier an je einem Wirbelkörper 24 befestigten Knochenfixationsmitteln 1 und einem Längsträger 11.

## Patentansprüche

1. Vorrichtung zur Verbindung eines Längsträgers (11) mit einem Knochen oder Knochenfragment umfassend
A) ein Knochenfixationsmittel (1) mit einer Zentralachse (5), einem koaxial angeordneten Unterteil (3) zur Fixation mit einem Knochen und einem koaxial angeordneten Oberteil (2) mit einem oberen Ende (13) und einem unteren Ende (14); wobei
B) das Oberteil (2) einen am oberen Ende (13) des Oberteils (2) offenen Kanal (12) mit quer zur Längsachse (5) gerichteter Kanalachse (21) zur Aufnahme eines Längsträgers (11) umfasst;
C) das Oberteil (2) zwischen seinem unteren Ende (14) und dem Kanal (12) einen zur Zentralachse (5) parallelen Schlitz (18) umfasst, welcher das Oberteil (2) radial durchdringt, so dass zwei quer zur Kanalachse (21) elastisch deformierbare Zungen (19) gebildet werden; und
D) die Vorrichtung einen Klemmring (15) mit einer einen Durchmesser d aufweisenden Zentralbohrung (22) umfasst, welcher in eine erste Position verschiebbar ist, in welcher die beiden Zungen (19) gegen die Kanalachse (21) pressbar sind, so dass ein im Kanal (12) eingelegter Längsträger (11) in der Vorrichtung lösbar blockierbar ist,
**dadurch gekennzeichnet, dass**
E) das Oberteil (2) zwischen seinem unteren Ende (14) und dem Kanal (12) eine zur Zentralachse (5) konzentrische Nute (8) mit einem Kerndurchmesser D_{K1} umfasst, wobei der Kerndurchmesser D_{K1} der Nute (8) kleiner als der Durchmesser d der Zentralbohrung (22) im Klemmring (15) ist, so dass der Klemmring (15) in eine zweite Position verschiebbar ist, in welcher die beiden Zungen (19) bezüglich der Zentralachse (5) elastisch spreizbar sind; und
F) das Oberteil (2) zwischen der Nute (8) und dem Kanal (12) einen zur Zentralachse (5) konzentrischen, kreisringförmigen Absatz (10) mit einem Aussendurchmesser D_{A} umfasst, wobei D_{A} > d ist.

2. Vorrichtung nach Anspruch 1, **dadurch gekennzeichnet, dass** das Oberteil (2) an seinem unteren Ende (14) einen Durchmesser D aufweist, wobei D ≥ d ist.

3. Vorrichtung nach Anspruch 1 oder 2, **dadurch gekennzeichnet, dass** das Oberteil (2) zwischen der Nute (8) und dem Kanal (12) eine zweite, zur Zentralachse (5) konzentrische, kreisförmige Nute (7) auf, welche einen Kerndurchmesser D_{K2} ≥ d aufweist, so dass der Klemmring (15) in seiner ersten Position in der zweiten Nute (7) ist.

4. Vorrichtung nach Anspruch 3, **dadurch gekennzeichnet, dass** das Verhältnis des Kerndurchmessers D_{K2} dieser zweiten Nute (7) zum Durchmesser d der Zentralbohrung (22) im Klemmring (15) zwischen 102% und 100% beträgt, so dass der Klemmring (15) in seiner ersten Position axial in der zweiten Nute (7) arretierbar ist.

5. Vorrichtung nach Anspruch 4, **dadurch gekennzeichnet, dass** das Verhältnis des Kerndurchmessers D_{K2} der zweiten Nute (7) zum Durchmesser d der Zentralbohrung (22) im Klemmring (15) zwischen 100,5% und 100% beträgt.

6. Vorrichtung nach einem der Ansprüche 1 bis 5, **dadurch gekennzeichnet, dass** der Klemmring (15) ein oberes Ende (16) aufweist und dass sich die Zentralbohrung (22) im Klemmring (15) gegen das obere Ende (16) konisch oder sphärisch erweitert.

7. Vorrichtung nach einem der Ansprüche 1 bis 6, **dadurch gekennzeichnet, dass** der Klemmring (15) ein unteres Ende (17) aufweist und dass die Zentralbohrung (22) im Klemmring (15) scharfkantig in das untere Ende (17) des Klemmringes (15) mündet.

8. Implantat mit einer Vorrichtung nach einem der Ansprüche 1 bis 7, **dadurch gekennzeichnet, dass**
a) dass die Vorrichtung zusätzlich einen stabförmigen Längsträger (11) mit einem Durchmesser D_{L} umfasst;
b) der Kanal (6) im undeformierten Zustand der Zungen (10) eine Lichte Weite L_{W} aufweist; und
c) das Verhältnis von L_{W} / D_{L} zwischen 90% und 110% beträgt.

9. Implantat nach Anspruch 8, **dadurch gekennzeichnet, dass** das Verhältnis L_{W} / D_{L} zwischen 98% und 102% beträgt.

10. Implantat nach Anspruch 9, **dadurch gekennzeichnet, dass** das Verhältnis L_{W} / D_{L} zwischen 99% und 101% beträgt.

## Claims

1. Device for connecting a longitudinal member (11) to a bone or bone fragment, the device comprising:
A) a bone fixation means (1) having a longitudinal axis (5), a coaxially disposed lower part (3) for fixation to the bone, and a coaxially disposed upper part (2) having an upper end (13) and a lower end (14); wherein
B) the upper part (2) includes a channel (12) having an opening at the upper end (13) of the upper part (2) for accommodating the longitudinal member (11), and a channel axis (21) extending transverse to the longitudinal axis (5);
C) the upper part (2) further including, between the lower end (14) of the upper part (2) and the channel (12), a slit (18) which extends parallel to the longitudinal axis (5) and which passes radially through the upper part (2) forming two elastically deformable sidewalls (19); and wherein
D) the device further comprises a locking ring (15) having a central borehole (22) having a diameter d, the locking ring (15) being moveable from a first position, wherein the two sidewalls (19) are elastically displaceable with respect to the longitudinal axis (5), to a second position, wherein the two sidewalls (19) are pressed against the channel axis (21) so that the longitudinal member (11) placed within the channel (12) is locked within the channel (12);
**characterized in that**
D) the upper part (2), between the lower end (14) of the upper part (2) and the channel (12) further comprises a groove (8), which is concentric with the longitudinal axis (5) and has a diameter D_{K1}, wherein the diameter D_{K1} of the groove (8) is smaller than the diameter d of the central borehole (22) formed in the locking ring (15) so that the locking ring (15) permits the two sidewalls (1) to be elastically displaceable with respect to the longitudinal axis (5) when in the first position; and wherein
E) the upper part (2), between the groove (8) and the channel (12), further comprises a shoulder (10), concentric with the longitudinal axis (5), and having an external diameter D_{A}, wherein the diameter D_{A} is greater than the diameter d.

2. The device of claim 1, wherein the lower end (14) of the upper part (2) has a diameter D, wherein the diameter D is greater than or equal to the diameter d.

3. The device of claims 1 or 2, wherein the upper part (2) further comprises a second groove (7) located between the first groove (8) and the channel (12), the second groove (7) being concentric with the longitudinal axis (5) and having a diameter D_{K2}, wherein the diameter D_{K2} is greater than or equal to the diameter d so that the locking ring (15) is aligned with the second groove (7) when in the second position.

4. The device of claim 3. wherein the ratio of the diameter D_{K2} of the second groove (7) to the diameter d of the central borehole (22) formed in the locking ring (15) is between 102% and 100% so that the locking ring (15) is axially locked when being in its first position.

5. The device of claim 4, **characterized in that** the ratio of the diameter D_{K2} of the second groove (7) to the diameter d of the central borehole (22) formed in the locking ring (15) is between about 100.5% and about 100%.

6. The device of one of the claims 1 to 5, wherein the locking ring (15) includes an upper end (16), the central borehole (22) formed in the locking ring (15) expands conically or spherically towards the upper end (16).

7. The device of one of the claims 1 to 6, wherein the locking ring (15) further includes a lower end (17), the central borehole (22) formed in the locking ring (15) ending with a sharp edge in the lower end (17) of the locking ring (15).

8. Implant with a device of one of the claims 1 to 7, wherein
A) the device further comprises a longitudinal member (11) with a diameter D_{L};
B) the channel (6) has an interior width L_{W} in the undeformed position of the sidewalls (10); and
C) the ratio of L_{W} to D_{L} is between about 90% and about 110%.

9. The implant of claim 8, wherein the ratio of L_{W} to D_{L} is between about 98% and about 102%.

10. The implant of claim 9, wherein the ratio of L_{W} to D_{L} is between about 99% and about 101%.

## Revendications

1. Dispositif permettant d'assembler un support longitudinal (11) à un os ou à un fragment d'os et comprenant
A) un moyen de fixation d'ostéosynthèse (1) avec un axe central (5), une partie inférieure (3) disposée de manière coaxiale et destinée à être fixée à un os ainsi qu'une partie supérieure (2) disposée de manière coaxiale et comportant une extrémité supérieure (13) et une extrémité inférieure (14);
B) la partie supérieure (2) comprenant un canal (12) ouvert à l'extrémité supérieure (13) de la partie supérieure (2), lequel présente un axe de canal (21) orienté transversalement à l'axe longitudinal (5) et qui sert à recevoir un support longitudinal (11);
C) la partie supérieure (2) comprenant, entre son extrémité inférieure (14) et le canal (12), une fente (18) parallèle à l'axe central (5), laquelle traverse radialement la partie supérieure (2) de sorte à former deux languettes (19) déformables de manière élastique transversalement à l'axe de canal (21); et
D) le dispositif comprenant une bague de blocage (15) avec un trou central (22) présentant un diamètre d qu'il est possible de déplacer dans une première position dans laquelle les deux languettes (19) peuvent être pressées en direction de l'axe de canal (21), de sorte qu'un support transversal (11) placé dans le canal (12) peut être bloqué de manière amovible dans ledit dispositif,
**caractérisé en ce que**
E) la partie supérieure (2) comprend, entre son extrémité inférieure (14) et le canal (12), une rainure (8) concentrique à l'axe central (5) présentant un diamètre de noyau D_{K1}, le diamètre de noyau D_{K1} de la rainure (8) étant inférieur au diamètre d du trou central (22) ménagé dans la bague de blocage (15), de sorte qu'il est possible de déplacer ladite bague de blocage (15) dans une deuxième position dans laquelle les deux languettes (19) peuvent s'écarter de manière élastique par rapport à l'axe central (5); et
F) la partie supérieure (2) comprend, entre la rainure (8) et le canal (12), une saillie (10) en forme d'anneau circulaire, concentrique à l'axe central (5), laquelle présente un diamètre extérieur D_{A}, D_{A} étant supérieur à d (D_{A} > d).

2. Dispositif selon la revendication 1, **caractérisé en ce que** la partie supérieure (2) présente à son extrémité inférieur (14) un diamètre D, D étant supérieur ou égal à d (D ≥ d).

3. Dispositif selon la revendication 1 ou 2, **caractérisé en ce que** la partie supérieure (2) comprend, entre la rainure (8) et le canal (12), une deuxième rainure circulaire (7), concentrique à l'axe central (5), laquelle présente un diamètre de noyau D_{K2} ≥ d, de sorte que la bague de blocage (15), lorsqu'elle est dans sa première position, se trouve dans la deuxième rainure (7).

4. Dispositif selon la revendication 3, **caractérisé en ce que** le rapport entre le diamètre de noyau Dₖ₂ de cette deuxième rainure (7) et le diamètre d du trou central (22) ménagé dans la bague de blocage (15) est compris entre 102% et 100%, de sorte qu'il est possible de bloquer axialement la bague de blocage (15) dans la deuxième rainure (7) lorsque elle se trouve dans sa première position.

5. Dispositif selon la revendication 4, **caractérisé en ce que** le rapport entre le diamètre de noyau Dₖ₂ de la deuxième rainure (7) et le diamètre d du trou central (22) ménagé dans la bague de blocage (15) est compris entre 100,5% et 100%.

6. Dispositif selon l'une des revendications 1 à 5, **caractérisé en ce que** la bague de blocage (15) présente une extrémité supérieure (16), et **en ce que** le trou central (22) ménagé dans la bague de blocage (15) va en s'élargissant de manière conique ou sphérique vers l'extrémité supérieure (16).

7. Dispositif selon l'une des revendications 1 à 6, **caractérisé en ce que** la bague de blocage (15) présente une extrémité inférieure (17), et **en ce que** le trou central (22) ménagé dans la bague de blocage (15) débouche dans l'extrémité inférieure (17) de la bague de blocage (15) en présentant une arête vive.

8. Implant avec un dispositif selon l'une des revendications 1 à 7, **caractérisé en ce que**
a) le dispositif comprend en outre un support longitudinal (11) en forme de tige présentant un diamètre D_{L};
b) le canal (6) présente, lorsque les languettes (10) sont à l'état non-déformé, une largeur intérieure L_{W}; et
c) le rapport L_{W}/D_{L} est compris entre 90% et 110%.

9. Implant selon la revendication 8, **caractérisé en ce que** le rapport L_{W}/D_{L} est compris entre 98% et 102%.

10. Implant selon la revendication 9, **caractérisé en ce que** le rapport L_{W}/D_{L} est compris entre 99% et 101%.
